# EUROPÄISCHE PATENTSCHRIFT

(11) **EP 2 291 341 B1**
(45) Veröffentlichungstag und Bekanntmachung des Hinweises auf die Patenterteilung: **28.03.2012**
(21) Anmeldenummer: 09749856.2
(22) Anmeldetag: 20.05.2009
(51) Int. Cl.: C07C 2/76

(54) **VERFAHREN ZUR ERZEUGUNG VON BENZOL, TOLUOL (UND NAPHTHALIN) AUS C1-C4-ALKANEN UNTER ÖRTLICH GETRENNTER KO-DOSIERUNG VON WASSERSTOFF**
METHOD FOR PRODUCING BENZENE, TOLUENE (AND NAPTHALENE) FROM C1-C4 ALKANES UNDER LOCAL SEPARATE CO-DOSING OF HYDROGEN
PROCÉDÉ DE PRODUCTION DE BENZÈNE, DE TOLUÈNE (ET DE NAPHTALINE) À PARTIR D ALCANES C1-C4 AVEC DOSAGE CONJOINT LOCALEMENT SÉPARÉ D HYDROGÈNE

(30) Priorität: 21.05.2008 EP 08156624
(43) Veröffentlichungstag der Anmeldung: 09.03.2011
(73) Patentinhaber: BASF SE, 67056 Ludwigshafen (DE)
(72) Erfinder: KIEßLICH, Frank, 63128 Dietzenbach (DE); GRITSCH, Achim, 70197 Stuttgart (DE); SCHNEIDER, Christian, 68165 Mannheim (DE); KOSTOVA, Albena, 68163 Mannheim (DE)
(86) Internationale Anmeldenummer: PCT/EP2009/056104
(87) Internationale Veröffentlichungsnummer: WO 2009/141366

(56) Entgegenhaltungen:
- WO-A-03/000826
- WO-A-2007/144324
- US-A1- 2007 249 879

## Beschreibung

Die vorliegende Erfindung betrifft ein Verfahren zur nicht-oxidativen Dehydroaromatisierung eines C₁-C₄-Aliphaten enthaltenden Eduktstroms durch Umsetzen des Eduktstroms in Gegenwart eines Katalysators in einer Reaktionszone 1 zu einem aromatische Kohlenwasserstoffe enthaltenden Produktstrom P und Regenerierung des durch abgelagerten Koks in seiner Aktivität geminderten Katalysators mit einem Wasserstoff enthaltenden Gemisch H in einer Reaktionszone 2, wobei mindestens ein Teil des abgelagerten Koks in Methan umgewandelt wird und mindestens ein Teil des entstandenen Methans der Reaktionszone 1 zugeführt wird.

Aromatische Kohlenwasserstoffe wie Benzol, Toluol, Ethylbenzol, Styrol, Xylol und Naphthalin stellen bedeutende Zwischenprodukte in der chemischen Industrie dar, deren Bedarf nach wie vor steigt. In der Regel werden sie durch katalytische Reformierung aus Naphtha gewonnen, das seinerseits aus Erdöl erhalten wird. Neuere Untersuchungen zeigen, dass die weltweiten Erdölvorräte im Vergleich zu den Erdgasvorräten stärker begrenzt sind. Daher ist die Herstellung von aromatischen Kohlenwasserstoffen aus Edukten, die aus Erdgas gewonnen werden können, eine inzwischen auch wirtschaftlich interessante Alternative. Die Hauptkomponente von Erdgas stellt üblicherweise Methan dar.

Eine mögliche Reaktionsroute zur Gewinnung von Aromaten aus Aliphaten stellt die nicht-oxidative Dehydroaromatisierung (DHAM) dar. Die Umsetzung erfolgt hierbei unter nicht-oxidativen Bedingungen, insbesondere unter Ausschluss von Sauerstoff. Bei der DHAM findet eine Dehydrierung und Cyclisierung der Aliphaten zu den entsprechenden Aromaten unter Freisetzung von Wasserstoff statt.

Ein großes Problem für die technische Anwendung der Dehydroaromatisierung unter nicht-oxidativen Bedingungen stellt die Verkokung dar, da sie in relativ kurzer Zeit die Aktivität des Katalysators herabsetzt, was zu kurzen Produktionszyklen und einem hohen Regenerierungsbedarf führt. Häufig geht mit der Verkokung zudem eine verkürzte Lebensdauer des Katalysators einher. Auch die Regenerierung der Katalysatoren ist nicht unproblematisch, da für ein wirtschaftliches Verfahren zum einen regelmäßig die Ausgangsaktivitäten wieder herstellbar sein müssen und zum anderen dies über eine große Anzahl von Zyklen möglich sein muss.

Die Koksablagerungen wirken sich zudem ungünstig auf die Stoffbilanz bzw. die Ausbeute aus, da jedes Molekül Edukt, das in Koks umgewandelt wird, nicht mehr für die gewünschte Reaktion zu Aromaten zur Verfügung steht. Die bisher im Stand der Technik erreichten Koksselektivitäten liegen in den meisten Fällen bei über 20 % bezogen auf den umgesetzten Aliphaten.

Eine weitere Schwierigkeit bei der technischen Durchführung der DHAM liegt im Einbringen der benötigen Reaktionswärme. Die DHAM ist eine endotherme Reaktion, die auf externe Wärmezufuhr angewiesen ist. Wird die Reaktion indirekt geheizt, sind gro-βe Wärmeaustauscherflächen erforderlich, die das Verfahren apparativ und wirtschaftlich aufwändig gestalten. Darüber hinaus finden an den Wärmeaustauscherflächen aufgrund der höheren Temperaturen unerwünschte Nebenreaktionen statt, beispielsweise Verkokung.

WO-A 03/000826 beschreibt ein Verfahren zur Aromatisierung von Methan, bei dem das Methan in einer Reaktionszone in Gegenwart eines aktiven Katalysators umgesetzt wird, wobei der Katalysator deaktiviert wird. Ein Teil des deaktivierten Katalysators wird in einer Regenerierungszone mit einem Regenerierungsgas regeneriert, wobei der Katalysator zwischen der Reaktionszone und der Regenerierungszone zirkuliert. Als Regenerierungsgase können Sauerstoff oder Luft, Wasserstoff und Wasserdampf eingesetzt werden. Die bei der Regenerierung entstehenden Gase werden nicht weiter verwendet. Die bei der Regenerierung entstehende Wärme wird durch den Katalysator selbst oder aber andere Wärmeaustauschermedien in die Reaktionszone überführt.

US-A 2007/0249879 betrifft ein Verfahren zur Umsetzung von Methan in höhere Kohlenwasserstoffe einschließlich Aromaten. Der verwendete Reaktor besteht aus mindestens zwei in Reihe geschalteten Reaktionszonen. Der teilchenförmig vorliegende Katalysator wird von der ersten in die zweite Reaktionszone geführt, der methanhaltige Strom in umgekehrte Richtung von der zweiten in die erste Reaktionszone. In allen Reaktionszonen findet eine Umsetzung des Methans zu Produkt statt. Teile des Katalysators können zur Regenerierung entnommen werden und nach der Regenerierung zurückgeführt werden. Die Regenerierung erfolgt mittels eines sauerstoffhaltigen Gases. Der Katalysator wird gegebenenfalls anschließend mit einem wasserstoffhaltigen Gas aktiviert. Zur Einführung von Wärme in das Reaktionssystem kann ein Teil des Katalysators entnommen werden und in einer separaten Heizzone mit Verbrennungsgasen, die von einer zusätzlichen Brennstoffquelle stammen, aufgeheizt werden. Der aufgeheizte Katalysator wird dann in die Reaktionszone zurückgeführt.

In US-A 2007/0249880 wird ein Verfahren zur Umsetzung von Methan zu aromatischen Kohlenwasserstoffen in Gegenwart eines Katalysators offenbart, wobei die Reaktionszone mit einem inversen Temperaturprofil gefahren wird. Auch hier kann der Katalysator nach Entnahme regeneriert bzw. auf Temperaturen oberhalb der Reaktionstemperatur mittels Verbrennungsgasen erhitzt werden und anschließend jeweils in die Reaktionszonen zurückgeführt werden.

WO-A 2006/011568 beschreibt ein Verfahren zur Herstellung von aromatischen Kohlenwasserstoffen und Wasserstoff. Hierzu wird ein Methan und 2 bis 10 % Wasserstoff enthaltender Gasstrom über einen Katalysator für die Dehydroaromatisierung geleitet. Die Zufuhr des Methans wird temporär unterbrochen. Gemäß den aufgeführten Beispielen wird nach 5 Stunden Reaktion (Zufuhr eines Methan/Wasserstoffgemisches) für zwei Stunden die Methanzufuhr abgestellt, so dass der Katalysator in Wasserstoffatmosphäre regeneriert wird.

Über die im Stand der Technik bekannten Verfahren hinaus besteht bedarf an weiteren, verbesserten Verfahren zur Herstellung von Aromaten aus C₁-C₄-Aliphaten, die eine hohe Ausbeute an aromatischen Kohlenwasserstoffen in Bezug auf die eingesetzten C₁-C₄-Aliphaten aufweisen, mit einer geringeren externen Energiezufuhr auskommen und geringere Wärmeaustauscherflächen benötigen.

Diese Aufgabe wird erfindungsgemäß gelöst durch ein Verfahren zur nicht-oxidativen Dehydroaromatisierung eines C₁-C₄-Aliphaten enthaltenden Eduktstroms E, umfassend die Schritte
I. Umsetzen des Eduktstroms E unter nicht-oxidativen Bedingungen in Gegenwart eines Katalysators in einer Reaktionszone 1 zu einem aromatische Kohlenwasserstoffe enthaltenden Produktstrom P,
II. Regenerierung des durch abgelagerten Koks in seiner Aktivität geminderten Katalysators aus Schritt I mit einem Wasserstoff enthaltenden Gasstrom H in einer Reaktionszone 2, wobei mindestens ein Teil des abgelagerten Koks in Methan umgewandelt wird und ein methanhaltiger Gasstrom M entsteht,
dadurch gekennzeichnet, dass mindestens ein Teil des bei der Regenerierung in der Reaktionszone 2 entstandenen Methans der Reaktionszone 1 zugeführt wird.

Bei der Regenerierung des deaktivierten Katalysators mit Wasserstoff entsteht aus den Koksablagerungen in einer exothermen Reaktion Methan. Dieses wird erfindungsgemäß der Reaktionszone 1 zugeführt und steht somit wieder als Edukt zur Verfügung. Dies führt zu einer Erhöhung der Gesamtausbeute an Aromaten bezogen auf die eingesetzte Menge C₁-C₄-Aliphaten. Gemäß einer Ausführungsform der vorliegenden Erfindung wird der bei der Regenerierung entstehende Gasstrom M ohne Abtrennung des Wasserstoffs in die Reaktionszone 1 zurückgeführt, eine aufwändige und teure Trennung der beiden sehr tief siedenden Verbindung Wasserstoff und Methan ist nicht notwendig. Die Beimengung von Wasserstoff im Eduktstrom beeinflusst die Verkokungsneigung des Katalysators zudem positiv. Gemäß einer weiteren Ausführungsform der vorliegenden Erfindung wird mindestens ein Teil des in Gasstrom M enthaltenen Wasserstoffs vor der Rückführung in Reaktionszone 1 zurückgeführt. Diese Ausführungsform besitzt den Vorteil, dass der Wasserstoffgehalt in der Reaktionszone 1 unabhängig von den Bedingungen in der Reaktionszone 2, von denen die Zusammensetzung des Gasstroms M abhängt, eingestellt werden kann.

Gemäß einer weiteren Ausführungsform der vorliegenden Erfindung wird die bei der Regenerierung des Katalysators erzeugte Wärme direkt durch die Rückführung des Katalysators und des Methans bzw. Gasstroms M in die Reaktionszone 1 überführt. Hierdurch wird ein Teil der für die Aromatisierung benötigten Reaktionswärme im System selbst erzeugt, was einen verringerten externen Energiebedarf des Gesamtsystems zur Folge hat.

Nicht-oxidativ gemäß der vorliegenden Erfindung bedeutet in Bezug auf die DHAM, dass die Konzentration von Oxidationsmitteln wie Sauerstoff oder Stickoxiden im Eduktstrom E unterhalb von 5 Gew.-%, bevorzugt unterhalb von 1 Gew.-%, besonders bevorzugt unterhalb von 0,1 Gew.-% liegt. Ganz besonders bevorzugt ist das Gemisch frei von Sauerstoff. Ebenfall besonders bevorzugt ist eine Konzentration an Oxidationsmitteln im Gemisch E, die gleich groß oder geringer ist als die Konzentration an Oxidationsmitteln in der Quelle, aus der die C₁-C₄-Aliphaten stammen.

In Bezug auf die Regenerierung bedeutet nicht-oxidativ im Rahmen der vorliegenden Erfindung, dass die aus der DHAM stammenden Koksablagerungen auf dem Katalysator zu dessen Regenerierung nicht mittels Oxidationsmitteln wie Luft oder Sauerstoff in CO und/oder CO₂ überführt werden. Insbesondere liegt die Konzentration an Oxidationsmitteln in dem zur Regenerierung in Schritt II einzusetzenden Gemisch H unterhalb von 5 Gew.-%, bevorzugt unterhalb von 1 Gew.-%, besonders bevorzugt unterhalb von 0,1 Gew.-%.

Die Konzentration an Methan in dem zur Regenerierung in Schritt II eingesetztem Wasserstoff enthaltenden Gasstrom H beträgt höchstens 70 Gew.-%, bevorzugt höchstens 50 Gew.-%, besonders bevorzugt höchstens 30 Gew.-% und besonders bevorzugt höchstens 15 Gew.-%.

Erfindungsgemäß enthält der Eduktstrom E mindestens einen Aliphaten mit 1 bis 4 Kohlenstoffatomen. Zu diesen Aliphaten gehören beispielsweise Methan, Ethan, Propan, n-Butan, i-Butan, Ethen, Propen, 1- und 2-Buten, Isobuten. In einer Ausführungsform der Erfindung enthält der Eduktstrom E mindestens 50 mol%, bevorzugt mindestens 60 mol%, besonders bevorzugt mindestens 70 mol%, außerordentlich bevorzugt mindestens 80 mol-%, insbesondere mindestens 90 mol% C₁-C₄-Aliphaten.

Unter den Aliphaten werden besonders bevorzugt die gesättigten Alkane verwendet, Eduktstrom E enthält dann bevorzugt mindestens 50 mol%, bevorzugt mindestens 60 mol%, besonders bevorzugt mindestens 70 mol% außerordentlich bevorzugt mindestens 80 mol%, insbesondere mindestens 90 mol% Alkane mit 1 bis 4 C-Atomen.

Unter den Alkanen sind Methan und Ethan bevorzugt, insbesondere Methan. Gemäß dieser Ausführungsform der vorliegenden Erfindung enthält der Eduktstrom E bevorzugt mindestens 50 mol%, bevorzugt mindestens 60 mol%, besonders bevorzugt mindestens 70 mol% außerordentlich bevorzugt mindestens 80 mol-%, insbesondere mindestens 90 mol-% Methan.

Bevorzugt wird als Quelle der C₁-C₄-Aliphaten Erdgas eingesetzt. Die typische Zusammensetzung von Erdgas sieht folgendermaßen aus: 75 bis 99 mol-% Methan, 0,01 bis 15 mol-% Ethan, 0,01 bis 10 mol% Propan, bis zu 6 mol% Butan, bis zu 30 mol% Kohlendioxid, bis zu 30 mol% Schwefelwasserstoff, bis zu 15 mol-% Stickstoff und bis zu 5 mol% Helium. Das Erdgas kann vor dem Einsatz in dem erfindungsgemäßen Verfahren nach dem Fachmann bekannten Methoden gereinigt und angereichert werden. Zur Reinigung gehört beispielsweise die Entfernung von gegebenenfalls im Erdgas vorhandenen Schwefelwasserstoff oder Kohlendioxid und weiterer, im anschlie-βenden Verfahren unerwünschten Verbindungen.

Die in dem Eduktstrom E enthaltenen C₁-C₄-Aliphaten können auch aus anderen Quellen stammen, beispielsweise bei der Erdölraffination angefallen sein. Die C₁-C₄-Aliphaten können auch regenerativ (z.B. Biogas) oder synthetisch (z.B. Fischer-Tropsch-Synthese) hergestellt worden sein.

Falls als C₁-C₄-Aliphaten-Quelle Biogas verwendet wird, kann der Eduktstrom E zusätzlich noch Ammoniak, Spuren von niederen Alkoholen und weitere, für Biogas typische Beimischungen enthalten.

In einer weiteren Ausführungsform des erfindungsgemäßen Verfahrens kann als Eduktstrom E LPG (Liquid Petroleum Gas) eingesetzt werden. Gemäß einer weiteren Ausführungsform des erfindungsgemäßen Verfahrens kann als Eduktstrom E LNG (Liquified Natural Gas) eingesetzt werden.

Dem Eduktstrom E kann zusätzlich Wasserstoff, Wasserdampf, Kohlenmonoxid, Kohlendioxid, Stickstoff sowie ein oder mehrere Edelgase beigemischt werden. Vorzugsweise enthält der Eduktstrom E Wasserstoff, bevorzugt 0,1 bis 10 Vol-% Wasserstoff, besonders bevorzugt 0,1 bis 5 Vol-% Wasserstoff. Gemäß einer besonders bevorzugten Ausführungsform der vorliegenden Erfindung wird dem Eduktstrom der bei der Regenerierung entstandene Gasstrom M zugemischt, der Methan und bei der Regenerierung nicht verbrauchten Wasserstoff enthält.

In Schritt I des erfindungsgemäßen Verfahrens findet die Umsetzung des Eduktstroms E unter nicht-oxidativen Bedingungen in Gegenwart eines Katalysators in einer Reaktionszone 1 zu einem aromatische Kohlenwasserstoffe enthaltenden Produktstrom P statt. Bei dieser Umsetzung handelt es sich um eine Dehydroaromatisierung, d.h. die in dem Eduktstrom E enthaltenen C₁-C₄-Aliphaten reagieren unter Dehydrierung und Cyclisierung zu den entsprechenden Aromaten, wobei Wasserstoff freigesetzt wird. Erfindungsgemäß wird die DHAM in Gegenwart geeigneter Katalysatoren durchgeführt. Generell können alle Katalysatoren, die die DHAM katalysieren, in Schritt I des erfindungsgemäßen Verfahrens eingesetzt werden. Üblicherweise enthalten die DHAM-Katalysatoren einen porösen Träger und mindestens ein darauf aufgebrachtes Metall. Der Träger enthält üblicherweise eine kristalline oder amorphe anorganische Verbindung.

Erfindungsgemäß enthält der Katalysator bevorzugt mindestens ein Metallosilikat als Träger. Bevorzugt werden als Träger Aluminiumsilikate eingesetzt. Ganz besonders bevorzugt werden erfindungsgemäß als Träger Zeolithe verwendet. Bei Zeolithen handelt es sich um Aluminiumsilikate, die bei ihrer Herstellung üblicherweise in der Natriumform anfallen. In der Na-Form wird die wegen des Austausches von 4-wertigen Si-Atomen gegen 3-wertige Al-Atomen im Kristallgitter vorhandene überschüssige negative Ladung durch Na-Ionen ausgeglichen. Statt allein Natrium kann der Zeolith zum Ladungsausgleich auch weitere Alkali- und/oder Erdalkaliionen enthalten. Erfindungsgemäß bevorzugt weist der in den Katalysatoren enthaltene mindestens eine Zeolith eine Struktur auf, die aus den Strukturtypen Pentasil und MWW ausgewählt ist und besonders bevorzugt aus den Strukturtypen MFI, MEL, Mischstrukturen aus MFI und MEL und MWW ausgewählt ist. Ganz besonders bevorzugt wird ein Zeolith des Typs ZSM-5 oder MCM-22 eingesetzt. Die Bezeichnungen der Strukturtypen der Zeolithe entsprechen den Angaben in W. M. Meier, D. H. Olson und Ch. Baerlocher, "Atlas of Zeolithe Structure Types", Elsevier, 3. Auflage, Amsterdam 2001. Die Synthese der Zeolithe ist dem Fachmann bekannt und kann beispielsweise ausgehend von Alkalialuminat, Alkalisilikat und amorphem SiO₂ unter hydrothermalen Bedingungen durchgeführt werden. Hierbei kann über organische Templat-Moleküle, über die Temperatur und weitere experimentelle Parameter die Art der gebildeten Kanalsysteme im Zeolithen gesteuert werden.

Die Zeolithe können neben Al weitere Elemente wie Ga, B, Fe oder In enthalten.

Vorzugsweise werden die bevorzugt als Träger verwendeten Zeolithe in der H-Form, in der die Zeolithe auch kommerziell erhältlich sind, eingesetzt.

Bei der Überführung von der Na-Form in die H-Form werden die im Zeolithen enthaltenden Alkali- und/oder Erdalkaliionen gegen Protonen ausgetauscht. Ein übliches und gemäß der vorliegenden Erfindung bevorzugtes Verfahren zum Überführen der Katalysatoren in die H-Form ist ein zweistufiger Prozess, bei dem die Alkali- und/oder Erdalkaliionen zunächst gegen Ammoniumionen ausgetauscht werden. Beim Erhitzen des Zeoliths auf etwa 400 bis 500°C zersetzt sich das Ammoniumion in flüchtigen Ammoniak und in das im Zeolithen verbleibende Proton.

Dazu wird der Zeolith mit einer NH₄-haltigen Mischung behandelt. Als NH₄-haltige Komponente der NH₄-haltigen Mischung wird ein Ammoniumsalz, ausgewählt aus der Gruppe Ammoniumchlorid, Ammoniumcarbonat, Ammoniumhydrogencarbonat, Ammoniumnitrat, Ammoniumphosphat, Ammoniumacetat, Ammoniumhydrogenphosphat, Ammoniumdihydrogenphosphat, Ammoniumsulfat und Ammoniumhydrogensulfat eingesetzt. Bevorzugt wird als NH₄-haltige Komponente Ammoniumnitrat verwendet.

Die Behandlung des Zeolithen mit der NH₄-haltigen Mischung erfolgt nach den bekannten, zum Ammoniumaustausch von Zeolithen geeigneten Methoden. Dazu zählt beispielsweise das Tränken, Tauchen oder Besprühen des Zeolithen mit einer Ammoniumsalzlösung, wobei die Lösung im Allgemeinen im Überschuss angewendet wird. Als Lösungsmittel werden vorzugsweise Wasser und Alkohole verwendet. Die Mischung enthält üblicherweise 1 bis 20 Gew.-% der eingesetzten NH₄-Komponente. Die Behandlung mit der NH₄-haltigen Mischung wird üblicherweise über einen Zeitraum von mehreren Stunden und bei erhöhten Temperaturen durchgeführt. Nach dem Einwirken der NH₄-haltigen Mischung auf den Zeolithen kann überschüssige Mischung entfernt und der Zeolith gewaschen werden. Anschließend wird der Zeolith bei 40 bis 150 °C für mehrere Stunden, üblicherweise 4 bis 20 Stunden getrocknet. Daran schließt sich die Kalzinierung des Zeolithen bei Temperaturen von 300 bis 700 °C, bevorzugt von 350 bis 650 °C und besonders bevorzugt von 500 bis 600 °C an. Die Dauer der Kalzinierung beträgt üblicherweise 2 bis 24 Stunden, bevorzugt 3 bis 10 Stunden, besonders bevorzugt 4 bis 6 Stunden.

In einer bevorzugten Ausführungsform der vorliegenden Erfindung werden als Träger Zeolithe, die erneut mit einer NH₄-haltigen Mischung behandelt und anschließend getrocknet wurden, eingesetzt. Die erneute Behandlung der Zeolithe mit der NH₄-haltigen Mischung erfolgt gemäß der vorstehenden Beschreibung.

Kommerziell erhältliche Zeolithe in der H-Form haben üblicherweise bereits einen ersten Ammoniumaustausch durch Behandeln mit einer NH₄-haltigen Mischung mit anschließendem Trocknen und Kalzinieren durchlaufen. Deshalb können erfindungsgemäß kommerziell erworbene, in der H-Form vorliegende Zeolithe als Träger a) eingesetzt werden, bevorzugt werden sie jedoch einer erneuten Behandlung mit einer NH₄-haltigen Mischungen unterzogen und gegebenenfalls kalziniert.

Üblicherweise enthält der DHAM-Katalysator mindestens ein Metall. Üblicherweise wird das Metall ausgewählt aus den Gruppen 3 bis 12 des Periodensystems der Elemente (IUPAC). Erfindungsgemäß bevorzugt enthält der DHAM-Katalysator mindestens ein Element ausgewählt aus den Übergangsmetallen der Hauptgruppen 6 bis 11. Besonders bevorzugt enthält der DHAM-Katalysator Mo, W, Mn, Tc, Re, Fe, Ru, Os, Co, Rh, Ir, Ni, Pd, Pt, Cu, Ag, Au. Insbesondere enthält der DHAM-Katalysator mindestens ein Element ausgewählt aus der Gruppe Mo, W, Re, Fe, Ru, Os, Co, Rh, Ir, Ni, Pd, Pt, Cu. Ganz besonders bevorzugt enthält der DHAM-Katalysator mindestens ein Element ausgewählt aus der Gruppe Mo, W und Re.

Erfindungsgemäß ebenfalls bevorzugt enthält der DHAM-Katalysator mindestens ein Metall als Aktivkomponente und mindestens ein weiteres Metall als Dotierung. Die Aktivkomponente wird erfindungsgemäß ausgewählt aus Mo, W, Re, Ru, Os, Rh, Ir, Pd, Pt. Die Dotierung wird erfindungsgemäß ausgewählt aus der Gruppe Cr, Mn, Fe, Co, Ni, Cu, V, Zn, Zr und Ga, bevorzugt aus der Gruppe Fe, Co, Ni, Cu. Erfindungsgemäß kann der DHAM-Katalysator mehr als ein Metall als Aktivkomponente und mehr als ein Metall als Dotierung enthalten. Diese werden jeweils aus den für die Aktivkomponente und die Dotierung angegebenen Metallen ausgewählt.

Das mindestens eine Metall wird erfindungsgemäß nasschemisch oder trockenchemisch auf den Träger aufgebracht.

Nasschemisch werden die Metalle in Form wässriger, organischer oder organischwässriger Lösungen ihrer Salze oder Komplexe durch Imprägnieren des Trägers mit der entsprechenden Lösung aufgebracht. Als Lösungsmittel kann auch überkritisches CO₂ dienen. Die Imprägnierung kann nach der incipient-wetness-Methode erfolgen, bei der das poröse Volumen des Trägers durch in etwa gleiches Volumen an Imprägnierlösung aufgefüllt wird und man - gegebenenfalls nach einer Reifung - den Träger trocknet. Man kann auch mit einem Überschuss an Lösung arbeiten, wobei das Volumen dieser Lösung größer ist als das poröse Volumen des Trägers. Hierbei wird der Träger mit der Imprägnierlösung gemischt und ausreichend lange gerührt. Weiterhin ist es möglich, den Träger mit einer Lösung der entsprechenden Metallverbindung zu besprühen. Es sind auch andere, dem Fachmann bekannte Herstellmethoden wie Ausfällen der Metallverbindungen auf dem Träger, Aufsprühen einer Metallverbindung enthaltenden Lösung, Soltränkung etc. möglich. Nach dem Aufbringen des mindestens einen Metalls auf den Träger wird der Katalysator bei etwa 80 bis 130°C üblicherweise 4 bis 20 Stunden im Vakuum oder an Luft getrocknet.

Erfindungsgemäß kann das mindestens eine Metall auch auf trockenchemischem Wege aufgebracht werden, beispielsweise indem die bei höheren Temperaturen gasförmigen Metallcarbonyle wie Mo(CO)₆, W(CO)₆ und Re₂(CO)₁₀ aus der Gasphase auf dem Träger abgeschieden werden. Das Abscheiden der Metallcarbonylverbindung wird im Anschluss an das Kalzinieren des Trägers durchgeführt.

Erfindungsgemäß enthält der Katalysator 0,1 bis 20 Gew.-%, bevorzugt 0,2 bis 15 Gew.-%, besonders bevorzugt 0,5 bis 10 Gew.-%, jeweils bezogen auf das Gesamtgewicht des Katalysators des mindestens einen Metalls. Der Katalysator kann nur ein Metall enthalten, er kann ein Gemisch aus zwei, drei oder mehr Metallen enthalten. Die Elemente können nasschemisch gemeinsam in einer Lösung aufgebracht werden oder in verschiedenen Lösungen nacheinander mit Trocknungsschritten zwischen den einzelnen Auftragungen. Die Elemente können auch gemischt aufgebracht werden, d.h. ein Teil nasschemisch und ein anderer Teil trockenchemisch. Zwischen den Auftragungen der Metallverbindungen kann nach Bedarf entsprechend der vorstehenden Beschreibung kalziniert werden.

Erfindungsgemäß kann der Katalysator mindestens ein Metall aus der Gruppe der Aktivkomponente in Verbindung mit mindestens einem Metall ausgewählt aus der Gruppe der Dotierung enthalten. In diesem Fall liegt die Konzentration der Aktivkomponente bei 0,1 bis 20 Gew.-%, bevorzugt 0,2 bis 15 Gew.-%, besonders bevorzugt bei 0,5 bis 10 Gew.-%, jeweils bezogen auf das Gesamtgewicht des Katalysators.

Die Dotierung liegt in diesem Fall im Katalysator erfindungsgemäß in einer Konzentration von mindestens 0,1 Gew.-%, bevorzugt mindestens 0,2 Gew.-%, ganz besonders bevorzugt mindestens 0,5 Gew.-%, bezogen auf das Gesamtgewicht des Katalysators vor.

Gemäß einer weiteren bevorzugten Ausführungsform der vorliegenden Erfindung wird der Katalysator mit einem Bindemittel vermischt. Als Bindemittel eignen sich die üblichen, dem Fachmann bekannten Bindemittel wie Aluminiumoxid- und/oder Si-haltige Bindemittel. Besonders bevorzugt sind dabei Si-haltige Bindemittel; insbesondere eignen sich Tetraalkoxysilane, Polysiloxane und kolloidale SiO₂-Sole.

Erfindungsgemäß erfolgt nach Zugabe des Bindemittels ein Formgebungsschritt, in dem die Katalysatormasse gemäß den dem Fachmann bekannten Verfahren zu Formkörpern verarbeitet werden. Als formgebende Verfahren sind dabei beispielsweise Versprühen einer den Träger a) bzw. die Katalysatormasse enthaltenden Suspension, Sprühtrocknung, Tablettieren, Verpressen im feuchten oder trockenen Zustand und Extrudieren zu nennen. Zwei oder mehrere dieser Verfahren können auch kombiniert werden. Für das Verformen können Hilfsmittel wie Porenbildner und Anteigungsmittel oder auch andere, dem Fachmann bekannte Zusatzstoffe eingesetzt werden. Mögliche Anteigungsmittel sind solche Verbindungen, die zur Verbesserung der Misch-, Knet- und Fließeigenschaften führen. Vorzugsweise sind dies im Rahmen der vorliegenden Erfindung organische, insbesondere hydrophile Polymere wie beispielsweise Cellulose, Cellulosederivate wie Methylcellulose, Stärke wie Kartoffelstärke, Tapetenkleister, Acrylate, Polyacrylate, Polymethacrylate, Polyvinylalkohole, Polyvinylpyrrolidon, Polyisobutylen, Polytetrahydrofuran, Polyglykolether, Fettsäureverbindungen, Wachsemulsionen, Wasser oder Mischungen aus zwei oder mehr dieser Verbindungen. Als Porenbildner sind im Rahmen der vorliegenden Erfindung beispielsweise in Wasser oder wässrigen Lösungsmittelgemischen dispergier-, suspendier- oder emulgierbare Verbindungen wie Polyalkylenoxide, Polystyrol, Polyacrylate, Polymethacrylate, Polyolefine, Polyamide, Polyester, Kohlenhydrate, Cellulose, Cellulosederivate wie beispielsweise Methylcellulose, Zuckernaturfasern, Pulp, Graphit oder Mischungen aus zwei oder mehr dieser Verbindungen zu nennen. Porenbildner und/oder Anteigungsmittel werden nach der Verformung bevorzugt durch mindestens einen geeigneten Trocknungs- und/oder Kalzinierungsschritt aus dem erhaltenen Formkörper entfernt. Die dazu erforderlichen Bedingungen können analog zu den vorstehend für Kalzinierung beschriebenen Parametern gewählt werden und sind dem Fachmann bekannt.

Insbesondere für den Einsatz als Wirbelschichtkatalysatoren werden die Katalysatorformkörper mittels Sprühtrocknung hergestellt.

Die Geometrie der erfindungsgemäß erhältlichen Katalysatoren kann beispielsweise kugelförmig (hohl oder voll), zylindrisch (hohl oder voll), ring-, sattel-, stern-, bienenwaben- oder tablettenförmig sein. Weiterhin kommen Extrudate beispielsweise in Strang-, Trilob-, Quatrolob, Stern- oder Hohlzylinderform in Frage. Weiterhin kann die zu formende Katalysatormasse extrudiert, kalziniert und die so erhaltenen Extrudate gebrochen und zu Split oder Pulver verarbeitet werden. Der Split kann in verschiedene Siebfraktionen getrennt werden.

Gemäß einer bevorzugten Ausführungsform der Erfindung wird der Katalysator als Formkörper oder Split eingesetzt.

Gemäß einer weiteren bevorzugten Ausführungsform wird der Katalysator als Pulver eingesetzt. Das Katalysatorpulver kann dabei Bindemittel enthalten, aber auch frei von Bindemittel vorliegen.

Wenn der erfindungsgemäße Katalysator ein Bindemittel enthält, liegt dies in einer Konzentration von 5 bis 80 Gew.-%, bezogen auf das Gesamtgewicht des Katalysators vor, bevorzugt von 10 bis 50 Gew.-%, besonders bevorzugt von 10 bis 30 Gew.%.

Es kann von Vorteil sein, den zur Dehydroaromatisierung von C₁-C₄-Aliphaten verwendeten Katalysator vor der eigentlichen Reaktion zu aktivieren.

Diese Aktivierung kann mit einem C₁-C₄-Alkan, wie z.B. Ethan, Propan, Butan oder einem Gemisch hiervon, vorzugsweise Butan, erfolgen. Die Aktivierung wird bei einer Temperatur von 250 bis 850°C, vorzugsweise bei 350 bis 650°C, und einem Druck von 0,5 bis 5 bar, vorzugsweise bei 0,5 bis 2 bar, durchgeführt. Üblicherweise liegt die GHSV (Gas Hourly Space Velocity) bei der Aktivierung bei 100 bis 4000 h⁻¹, vorzugsweise bei 500 bis 2000 h⁻¹.

Es ist aber auch möglich eine Aktivierung durchzuführen, indem der Eduktstrom E das C₁-C₄-Alkan, oder ein Gemisch hiervon, per se schon enthält oder das C₁-C₄-Alkan, oder ein Gemisch hiervon, dem Eduktstrom E zugesetzt wird. Die Aktivierung wird bei einer Temperatur von 250 bis 650°C, vorzugsweise bei 350 bis 550°C, und einem Druck von 0,5 bis 5 bar, vorzugsweise bei 0,5 bis 2 bar, durchgeführt.

In einer weiteren Ausgestaltungsform ist es auch möglich zusätzlich zu dem C₁-C₄-Alkan noch Wasserstoff beizufügen.

Gemäß einer bevorzugten Ausführungsform der vorliegenden Erfindung wird der Katalysator mit einem H₂ enthaltenden Gasstrom aktiviert, der zusätzlich Inertgase wie N₂, He, Ne und Ar enthalten kann.

Erfindungsgemäß wird die Dehydroaromatisierung von C₁-C₄-Aliphaten in Gegenwart eines Katalysators bei Temperaturen von 400 bis 1000 °C, bevorzugt von 500 bis 900°C, besonders bevorzugt von 600 bis 800°C, insbesondere von 700 bis 800 °C, bei einem Druck von 0.5 bis 100 bar, bevorzugt bei 1 bis 30 bar, besonders bevorzugt bei 1 bis 10 bar, insbesondere 1 bis 5 bar, durchgeführt. Gemäß der vorliegenden Erfindung wird die Umsetzung bei einer GHSV (Gas Hourly Space Velocity) von 100 bis 10 000 h⁻¹, vorzugsweise von 200 bis 3000 h⁻¹ durchgeführt.

Die Dehydroaromatisierung von C₁-C₄-Aliphaten gemäß Schritt I als auch die Regenerierung des durch Koksablagerungen deaktivierten Katalysators mit Wasserstoff gemäß Schritt II kann grundsätzlich in allen aus dem Stand der Technik bekannten Reaktortypen durchgeführt werden. Eine geeignete Reaktorform ist der Festbett-, Radialstrom-, Rohr- oder Rohrbündelreaktor. Bei diesen befindet sich der Katalysator als Festbett in einem Reaktionsrohr oder in einem Bündel von Reaktionsrohren. Ebenso können die Katalysatoren als Wirbelschicht, Wanderbett oder Fließbett in den entsprechenden, dafür geeigneten Reaktortypen eingesetzt werden und das erfindungsgemäße Verfahren mit den derart vorliegenden Katalysatoren durchgeführt werden.

Erfindungsgemäß kann der Katalysator unverdünnt oder mit Inertmaterial vermischt eingesetzt werden. Als Inertmaterial kann jedes Material dienen, das sich bei den in den Reaktionszonen herrschenden Reaktionsbedingungen inert verhält, d.h. nicht reagiert. Als Inertmaterial eignet sich besonders der undotierte, für den Katalysator eingesetzte Träger, aber auch inerte Zeolithe, Aluminiumoxid, Siliziumdioxid etc. Die Teilchengröße des Inertmaterials liegt im Bereich der Größe der Katalysatorteilchen. Das Inertmaterial dient erfindungsgemäß hauptsächlich als kostengünstiger Wärmeüberträger, um Wärmeenergie aus der Reaktionszone 2 oder gegebenenfalls nach Ausschleusen und Aufheizen in die Reaktionszone 1 einzubringen.

Bevorzugt gemäß der vorliegenden Erfindung liegt der Katalysator unverdünnt oder mit Inertmaterial vermischt in der Reaktionszone 1, in der Reaktionszone 2 oder in beiden Reaktionszonen als Fest-, Wander- oder Wirbelbett vor. Besonders bevorzugt liegt der Katalysator bzw. das Gemisch aus Katalysator und Inertmaterial in der Reaktionszone 1, in der Reaktionszone 2 oder in beiden Reaktionszonen als Wirbelschicht vor

Die C₁-C₄-Aliphaten werden erfindungsgemäß unter Freisetzung von H₂ zu Aromaten umgesetzt. Der Produktstrom P enthält daher mindestens einen aromatischen Kohlenwasserstoff ausgewählt aus der Gruppe Benzol, Toluol, Ethylbenzol, Styrol, Xylol und Naphthalin. Besonders bevorzugt enthält er Benzol und Toluol. Weiterhin enthält der Produktstrom nicht umgesetzte C₁-C₄-Aliphaten entstandenen Wasserstoff und die im Eduktstrom E enthaltenen Inertgase wie N₂, He, Ne, Ar, dem Eduktstrom E zugesetzte Stoffe wie H₂ sowie bereits in E vorhandene Verunreinigungen.

Die Regenerierung gemäß Stufe II in Reaktionszone 2 wird bei Temperaturen von 600 °C bis 1000 °C und besonders bevorzugt von 700 °C bis 900 °C und Drücken von 1 bar bis 30 bar, bevorzugt von 1 bar bis 15 bar und besonders bevorzugt von 1 bis 10 bar durchgeführt.

Gemäß einer bevorzugten Ausführungsform der vorliegenden Erfindung liegt die Temperatur am Eintritt in die Reaktionszone 2 über der Temperatur am Eintritt in die Reaktionszone 1. Bevorzugt liegt die Eintrittstemperatur in Reaktionszone 2 mindestens 50°C, bevorzugt mindestens 75°C, besonders bevorzugt mindestens 100°C über der Eintrittstemperatur in der Reaktionszone 1.

Erfindungsgemäß wird der für die DHAM in Schritt I eingesetzte Katalysator regelmäßig mit dem im Gasstrom H enthaltenen Wasserstoff in Schritt II regeneriert. Dabei wird mindestens ein Teil des abgelagerten Kokses in Methan umgewandelt. Es entsteht ein methanhaltiger Gasstrom M, der neben dem entstandenen Methan nicht umgesetzten Wasserstoff sowie schon in Gemisch H enthaltene Stoffe umfasst. Erfindungsgemäß wird mindestens ein Teil des bei der Regenerierung entstandenen Methans der Reaktionszone 1 zugeführt. Das Methan kann nach Abtrennung von dem Gasstrom M der Reaktionszone 1 zugeführt werden. Bevorzugt werden mindestens 50% des in Reaktionszone 2 entstandenen Methans, besonders bevorzugt mindestens 70%, insbesondere mindestens 90% des in Reaktionszone 2 entstandenen Methans der Reaktionszone 1 zugeführt. Ganz besonders bevorzugt wird das gesamte bei der Regenerierung entstandene Methan der Reaktionszone 1 zugeführt.

Gemäß einer bevorzugten Ausführungsform der vorliegenden Erfindung wird mindestens ein Teil des bei der Regenerierung entstandenen, methanhaltigen Gasstroms M der Reaktionszone 1 zugeführt. Der Gasstrom M kann ohne vorherige Abtrennung einer oder mehrerer Bestandteile der Reaktionszone 1 zugeführt werden, es können jedoch auch ein oder mehrere Bestandteile vor der Rückführung des Gasstroms M abgetrennt werden. Dadurch kann das CH₄/H₂ Verhältnis am Eingang der Reaktionszone 1 gezielt eingestellt werden. Bevorzugt wird vor der Rückführung mindestens eines Teils des Gasstroms M mindestens ein Teil des darin enthaltenen, nicht umgesetzten Wasserstoffs abgetrennt.

Das in Stufe II entstandene Methan bzw. der methanhaltige Gasstrom M kann der Reaktionszone 1 direkt zugeführt werden oder durch Beimengung des Methans bzw. des Gasstroms M zum Eduktstrom E zugeführt werden.

Bei der Reaktionszone 1 und der Reaktionszone 2 handelt es sich um zwei Reaktionszonen, die sich räumlich getrennt in einem Reaktor oder räumlich getrennt in verschiedenen Reaktoren befinden. Die Reaktionszone 1 und die Reaktionszone 2 werden dabei durch die in ihnen ablaufenden Reaktionen definiert. In der Reaktionszone 1 läuft die Umsetzung der im Eduktstrom E enthaltenen C₁-C₄-Aliphaten zu aromatischen Kohlenwasserstoffen ab, in der Reaktionszone 2 die Umwandlung des auf den deaktivierten Katalysator abgelagerten Koks mit Hilfe des im Gasstrom H enthaltenen Wasserstoffs zu Methan.

Reaktionszone 1 und Reaktionszone 2 können durch Änderung der Gasströme jeweils ineinander umgewandelt werden. Gemäß einer bevorzugten Ausführungsform der vorliegenden Erfindung wird die Reaktionszone 1 durch Verminderung des Eduktstroms E und Zufuhr des Gasstroms H in die Reaktionszone 2 umgewandelt. Verminderung des Eduktstroms E bedeutet, dass der Eduktstrom E höchstens 10 Vol-% in die Reaktionszone der zugeführten Gase ausmacht, bevorzugt höchstens 5 Vol%, und besonders bevorzugt höchstens 1 Vol-%. Insbesondere bevorzugt ist die vollständige Schließung der Zufuhr des Eduktstroms E.

Gemäß einer bevorzugten Ausführungsform der Erfindung wird Reaktionszone 2 durch Verminderung des Gasstroms H und Zufuhr des Eduktstroms E in Reaktionszone 1 umgewandelt. Verminderung des Gasstroms H bedeutet, dass der Gasstrom H höchstens 10 Vol-% der in die Reaktionszone 2 zugeführten Gase bildet, bevorzugt höchstens 5 Vol-% und besonders bevorzugt höchstens 1 Vol-%, bezogen auf das Gesamtvolumen der Gaszufuhr. Besonders bevorzugt wird die Zufuhr des Gasstroms H vollständig geschlossen.

In einer weiteren Ausführungsform enthält der Eduktstrom E keinen Wasserstoff, dann kann die Zufuhr des Gasstroms H bei der Umwandlung der Reaktionszone 2 in die Reaktionszone 1 auch nur soweit gedrosselt werden, dass ein sich auf die Verkokung positiv auswirkender Gehalt an Wasserstoff in der Reaktionszone 1 eingestellt wird.

Besonders bevorzugt werden die Umwandlung der Reaktionszone 1 in die Reaktionszone 2 und die Umwandlung der Reaktionszone 2 in die Reaktionszone 1 mit einander gekoppelt im Wechsel durchgeführt, so dass eine Reaktionszone in zeitlichen Intervallen alternierend als Reaktionszone 1, in der die DHAM stattfindet, und als Reaktionszone 2, in der mindestens ein Teil des abgelagerten Kokses mit Hilfe von Wasserstoff in Methan umgewandelt wird, vorliegt. Die andere Reaktionszone liegt dazu jeweils zeitlich versetzt als Reaktionszone 2 und als Reaktionszone 1 vor. Erfindungsgemäß liegt die eine Reaktionszone von 1 bis 50 Stunden als Reaktionszone 1 (Dehydroaromatisierung) und von 1 bis 50 Stunden als Reaktionszone 2 (Regenerierung) vor.

Erfindungsgemäß können mehr als eine Reaktionszone 1 und mehr als eine Reaktionszone 2 vorhanden sein, es muss nur jeweils mindestens eine Reaktionszone 1 und mindestens eine Reaktionszone 2 vorliegen. Es können auch Reaktionszonen vorhanden sein, die sich in der Phase der Umwandlung von Reaktionszone 1 in Reaktionszone 2 befinden, weiterhin können Reaktionszonen vorliegen, in denen der Katalysator nach anderen Methoden regeneriert wird, beispielsweise mittels Sauerstoff oder Wasserdampf, wobei möglicherweise ein Recarbidisierungsschritt notwendig wird. Erfindungsgemäß bevorzugt liegen nur Reaktionszonen 1 und die Reaktionszonen 2 vor.

Zur Regenerierung des durch Koksablagerungen deaktivierten Katalysators aus Schritt I wird dieser erfindungsgemäß regelmäßig in der Reaktionszone 2 mit Wasserstoff regeneriert. Gemäß einer Ausführungsform der Erfindung wird der Katalysator dazu aus der Reaktionszone 1 in die Reaktionszone 2 überführt und dort mit Hilfe des Wasserstoff enthaltenden Gasstroms H regeneriert. Der regenerierte Katalysator wird dann wieder in die Reaktionszone 1 zurückgeführt. Gemäß einer weiteren Ausführungsform der Erfindung wird die Reaktionszone 1 wie vorstehend beschrieben durch Verminderung der Zufuhr des Eduktstroms E und Zufuhr des Gasstroms H in die Reaktionszone 2 umgewandelt, der deaktivierte Katalysator wird regeneriert und die Reaktionszone 2 anschließend wieder wie vorstehend beschrieben in die Reaktionszone 1 umgewandelt.

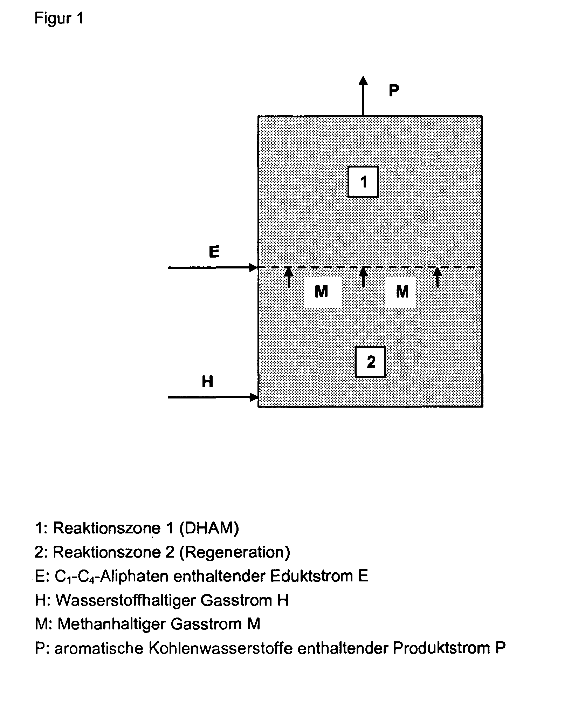

Gemäß einer besonders bevorzugten Ausführungsform der vorliegenden Erfindung liegen die Reaktionszone 1 und die Reaktionszone 2 räumlich getrennt in einem Reaktor vor. Der Reaktor enthält den Katalysator oder eine Mischung aus Katalysator und Inertmaterial in Form von Teilchen und wird als stationäres Wirbelbett betrieben, dabei handelt es sich um eine Blasen bildende oder turbulente Wirbelschicht, die mit einer geeigneten Vorrichtung zum Zurückhalten des Katalysators und gegebenenfalls des Inertmaterials versehen ist. Die Katalysatorteilchen oder die Mischung aus Katalysator und Inertmaterial sind dabei genügend fluidisiert, um die verschiedenen räumlichen Zonen des Reaktors regelmäßig zu durchlaufen. Die Zufuhr des Eduktstroms E erfolgt dabei oberhalb der Zufuhr des Gasstroms H. Diese Ausführungsform ist schematisch in Figur 1 dargestellt. Im Bereich der Zufuhr des Gasstroms H befindet sich die Reaktionszone 2, in der erfindungsgemäß die Umsetzung der Koksablagerungen zu Methan gemäß Schritt II des vorliegenden Verfahrens stattfindet. Der dabei entstehende Gasstrom M steigt nach oben in die Reaktionszone 1, in der die im Eduktstrom E enthaltenen C₁-C₄-Aliphaten zu Aromaten umgesetzt werden. Die fluidisierten Katalysatorteilchen bzw. die Mischung aus Katalysator und Inertmaterial bewegen sich aus der Reaktionszone 2 in die Reaktionszone 1 und umgekehrt, wandern also zwischen der Reaktionszone 1 und der Reaktionszone 2 hin und her.

Gemäß einer bevorzugten Ausführungsform der vorliegenden Erfindung wird mindestens ein Teil der bei der Regenerierung des Katalysators in Schritt II in der Reaktionszone 2 entstehenden Wärme der Reaktionszone 1 zugeführt, um zumindest teilweise zur Deckung der in Schritt I für die DHAM benötigten Energie beizutragen. Die Wärmezufuhr kann dabei direkt oder indirekt erfolgen. Vorzugsweise wird die Wärme direkt zugeführt. Dazu wird bevorzugt mindestens ein Teil der bei der Regenerierung des Katalysators in Schritt II in der Reaktionszone 2 entstehenden Wärme direkt durch Überführung mindestens eines Teils des regenerierten Katalysators aus der Reaktionszone 2 der Reaktionszone 1 zugeführt. Der regenerierte Katalysator dient als Wärmeträger. Gemäß einer weiteren bevorzugten Ausführungsform wird mindestens ein Teil der bei der Regenerierung des Katalysators in Schritt II in der Reaktionszone 2 entstehenden Wärme direkt durch den Gasstrom M aus der Reaktionszone 2 der Reaktionszone 1 zugeführt.

Besonders bevorzugt wird mindestens ein Teil der bei der Regenerierung des Katalysators in Schritt II in der Reaktionszone 2 entstehenden Wärme direkt durch Überführen mindestens eines Teils des regenerierten Katalysators und mindestens eines Teils des Gasstroms M aus der Reaktionszone 2 der Reaktionszone 1 zugeführt.

Bei der vorstehend beschriebenen und in Figur 1 schematisch dargestellten bevorzugten Ausführungsform der Erfindung, bei der beide Reaktionszonen in einem Reaktor vorliegen, der als nicht austragendes Wirbelbett betrieben wird, wird die bei der Regenerierung in der Reaktionszone 2 bei Schritt II entstehende Wärme durch den Gasstrom M sowie durch die hin- und herwandernden fluidisierten Katalysatorteilchen der Reaktionszone 1 zugeführt.

Bei Umwandlung der Reaktionszone 1 in die Reaktionszone 2 und umgekehrt liegt der Katalysator bevorzugt als Festbett oder als stationäre Wirbelschicht vor. Der als Reaktionszone 1 betriebene Teil kühlt aufgrund der endothermen DHAM ab. Nach Umwandlung in die Reaktionszone 2 heizt sich diese Reaktionszone durch die exotherme Umwandlung der Koksablagerungen in Methan auf. Wenn diese Reaktionszone dann wieder in die Reaktionszone 1 umgewandelt wird, wird zumindest ein Teil der bei der Umsetzung der Koksablagerungen zu Methan entstandenen Wärme durch den aufgeheizten Katalysator in die Reaktionszone 1 überführt.

Gemäß einer weiteren bevorzugten Ausführungsform der vorliegenden Erfindung kann ein Teil der in der Reaktionszone 1 in Schritt I des vorliegenden Verfahrens benötigten Energie durch Aufheizen des Katalysators und gegebenenfalls des Inertmaterials indirekt, beispielsweise durch ein Wärmetauscherbündel in Reaktionszone 1, zugeführt werden.

Gemäß einer weiteren bevorzugten Ausführungsform der vorliegenden Erfindung wird ein Teil der in der Reaktionszone 1 in Schritt I des vorliegenden Verfahrens benötigten Energie durch
i) Ausführen mindestens eines Teils des in Reaktionszone 1 oder 2 befindlichen Katalysators und gegebenenfalls des Inertmaterials aus der Reaktionszone 1 oder 2,
ii) Aufheizen des ausgeführten Katalysators und gegebenenfalls des Inertmaterials auf eine Temperatur oberhalb der Temperatur in der Reaktionszone 1 und
iii) Rückführen des aufgeheizten Katalysators und gegebenenfalls des Inertmaterial in die Reaktionszone 1
zugeführt.

Der ausgeführte Katalysator wird dabei auf eine Temperatur aufgeheizt, die mindestens 50°C, bevorzugt mindestens 100°C und besonders bevorzugt mindestens 150°C oberhalb der Temperatur in der Reaktionszone 1 liegt. Das Aufheizen des ausgeführten Katalysators kann direkt oder indirekt erfolgen. Bevorzugt wird der ausgeführte Katalysator direkt beheizt, indem beispielsweise Verbrennungsgase durch den Katalysator geführt werden. Alternativ kann mit den Verbrennungsgasen ein Inertgas erhitzt werden, welches dann in direktem Kontakt den Katalysator aufheizt.

## Patentansprüche

1. Verfahren zur nicht-oxidativen Dehydroaromatisierung eines C₁-C₄-Aliphaten enthaltenden Eduktstroms E, umfassend die Schritte
I. Umsetzen des Eduktstroms E unter nicht-oxidativen Bedingungen in Gegenwart eines Katalysators in einer Reaktionszone 1 zu einem aromatische Kohlenwasserstoffe enthaltenden Produktstrom P,
II. Regenerierung des durch abgelagerten Koks in seiner Aktivität geminderten Katalysators aus Schritt I mit einem Wasserstoff enthaltenden Gasstrom H in einer Reaktionszone 2, wobei mindestens ein Teil des abgelagerten Koks in Methan umgewandelt wird und ein methanhaltiger Gasstrom M entsteht,
**dadurch gekennzeichnet, dass** mindestens ein Teil des bei der Regenerierung in der Reaktionszone 2 entstandenen Methans der Reaktionszone 1 zugeführt wird.

2. Verfahren nach Anspruch 1, **dadurch gekennzeichnet, dass** mindestens ein Teil des bei der Regenerierung entstandenen Gasstroms M der Reaktionszone 1 zugeführt wird.

3. Verfahren nach Anspruch 1 oder 2 **dadurch gekennzeichnet, dass** mindestens ein Teil der bei der Regenerierung des Katalysators in Schritt II in der Reaktionszone 2 entstehenden Wärme der Reaktionszone 1 zugeführt wird.

4. Verfahren nach einem der Ansprüche 1 bis 3, **dadurch gekennzeichnet, dass** der Katalysator mit einem Inertmaterial vermischt eingesetzt wird.

5. Verfahren nach einem der Ansprüche 1 bis 4, **dadurch gekennzeichnet, dass** mindestens ein Teil der bei der der Regenerierung des Katalysators in Schritt II in der Reaktionszone 2 entstehenden Wärme direkt durch Überführung mindestens eines Teils des regenerierten Katalysators aus der Reaktionszone 2 der Reaktionszone 1 zugeführt wird.

6. Verfahren nach einem der Ansprüche 1 bis 5, **dadurch gekennzeichnet, dass** mindestens ein Teil der bei der Regenerierung des Katalysators in Schritt II in der Reaktionszone 2 entstehenden Wärme direkt durch den Gasstrom M aus der Reaktionszone 2 der Reaktionszone 1 zugeführt wird.

7. Verfahren nach einem der Ansprüche 1 bis 6, **dadurch gekennzeichnet, dass** mindestens ein Teil der in Schritt I benötigten Wärme durch
i) Ausführen mindestens eines Teils des in Reaktionszone 1 oder 2 befindlichen Katalysators aus der Reaktionszone 1 oder 2,
ii) Aufheizen des ausgeführten Katalysators auf eine Temperatur oberhalb der Temperatur in der Reaktionszone 1 und
iii) Rückführen des aufgeheizten Katalysators in die Reaktionszone 1 zugeführt wird.

8. Verfahren nach einem der Ansprüche 1 bis 7, **dadurch gekennzeichnet, dass** die Temperatur am Eintritt in die Reaktionszone 2 über der Temperatur am Eintritt in die Reaktionszone 1 liegt.

9. Verfahren nach einem der Ansprüche 1 bis 8, **dadurch gekennzeichnet, dass** sich Reaktionszone 1 und Reaktionszone 2 räumlich getrennt in einem Reaktor befinden.

10. Verfahren nach einem der Ansprüche 1 bis 8, **dadurch gekennzeichnet, dass** sich Reaktionszone 1 und Reaktionszone 2 räumlich getrennt in verschiedenen Reaktoren befinden.

11. Verfahren nach einem der Ansprüche 1 bis 10, **dadurch gekennzeichnet, dass** die Reaktionszone 1 in zeitlichen Intervallen durch Verminderung des Eduktstroms E und Zufuhr des Gasstroms H in die Reaktionszone 2 umgewandelt wird.

12. Verfahren nach einem der Ansprüche 1 bis 11 **dadurch gekennzeichnet, dass** die Reaktionszone 2 in zeitlichen Intervallen durch Verminderung des Gasstroms H und Zufuhr des Eduktstroms E in die Reaktionszone 1 umgewandelt wird.

13. Verfahren nach einem der Ansprüche 1 bis 12, **dadurch gekennzeichnet, dass** der Katalysator in der Reaktionszone 1, in der Reaktionszone 2 oder in beiden Reaktionszonen als Wirbelschicht vorliegt.

14. Verfahren nach einem der Ansprüche 1 bis 13, **dadurch gekennzeichnet, dass** der Eduktstrom E mindestens 50 Mol.-% C₁-C₄-Aliphaten enthält.

15. Verfahren nach einem der Ansprüche 1 bis 14, **dadurch gekennzeichnet, dass** der Eduktstrom E 0,1 bis 10 Vol.-% Wasserstoff enthält.

16. Verfahren nach einem der Ansprüche 1 bis 15, **dadurch gekennzeichnet, dass** der Gasstrom H mindestens 50 Vol.-% Wasserstoff enthält.

17. Verfahren nach einem der Ansprüche 1 bis 16, **dadurch gekennzeichnet, dass** der Katalysator mindestens ein Aluminiumsilikat und mindestens ein Metall ausgewählt aus der Gruppe Mo, W, Mn und Re enthält.

18. Verfahren nach Anspruch 17, **dadurch gekennzeichnet, dass** der Katalysator mindestens ein weiteres Metall ausgewählt aus der Gruppe Cr, Mn, V, Zn, Zr, Ga, Cu, Ni, Co und Fe enthält.

## Claims

1. A process for nonoxidatively dehydroaromatizing a reactant stream E comprising C₁-C₄-aliphatics, comprising the steps of
I. converting the reactant stream E under nonoxidative conditions in the presence of a catalyst in a reaction zone 1 to a product stream P comprising aromatic hydrocarbons,
II. regenerating the catalyst whose activity has been reduced by deposited coke from step I with a hydrogen-comprising gas stream H in a reaction zone 2, which converts at least a portion of the deposited coke to methane and forms a methane-containing gas stream M,
which comprises feeding at least a portion of the methane formed in the regeneration in reaction zone 2 to reaction zone 1.

2. The process according to claim 1, wherein at least a portion of the gas stream M formed in the regeneration is fed to reaction zone 1.

3. The process according to claim 1 or 2, wherein at least a portion of the heat arising in the regeneration of the catalyst in step II in reaction zone 2 is fed to reaction zone 1.

4. The process according to any one of claims 1 to 3, wherein the catalyst is used mixed with an inert material.

5. The process according to any one of claims 1 to 4, wherein at least a portion of the heat arising in the regeneration of the catalyst in step II in reaction zone 2 is fed directly to reaction zone 1 by transferring at least a portion of the regenerated catalyst from reaction zone 2.

6. The process according to any one of claims 1 to 5, wherein at least a portion of the heat arising in the regeneration of the catalyst in step II in reaction zone 2 is fed directly to reaction zone 1 through the gas stream M from reaction zone 2.

7. The process according to any one of claims 1 to 6, wherein at least a portion of the heat required in step I is supplied by
i) discharging at least a portion of the catalyst present in reaction zone 1 or 2 from reaction zone 1 or 2,
ii) heating the discharged catalyst to a temperature above the temperature in reaction zone 1 and
iii) recycling the heated catalyst into reaction zone 1.

8. The process according to any one of claims 1 to 7, wherein the temperature on entry into reaction zone 2 is above the temperature on entry into reaction zone 1.

9. The process according to any one of claims 1 to 8, wherein reaction zone 1 and reaction zone 2 are present spatially separately in one reactor.

10. The process according to any one of claims 1 to 8, wherein reaction zone 1 and reaction zone 2 are present spatially separately in different reactors.

11. The process according to any one of claims 1 to 10, wherein reaction zone 1 is converted to reaction zone 2 at time intervals by reducing reactant stream E and supplying gas stream H.

12. The process according to any one of claims 1 to 11, wherein reaction zone 2 is converted to reaction zone 1 at time intervals by reducing gas stream H and supplying reactant stream E.

13. The process according to any one of claims 1 to 12, wherein the catalyst is present as a fluidized bed in reaction zone 1, in reaction zone 2 or in both reaction zones.

14. The process according to any one of claims 1 to 13, wherein reactant stream E comprises at least 50 mol% of C₁-C₄-aliphatics.

15. The process according to any one of claims 1 to 14, wherein reactant stream E comprises from 0.1 to 10% by volume of hydrogen.

16. The process according to any one of claims 1 to 15, wherein gas stream H comprises at least 50% by volume of hydrogen.

17. The process according to any one of claims 1 to 16, wherein the catalyst comprises at least one aluminosilicate and at least one metal selected from the group of Mo, W, Mn and Re.

18. The process according to claim 17, wherein the catalyst comprises at least one further metal selected from the group of Cr, Mn, V, Zn, Zr, Ga, Cu, Ni, Co and Fe.

## Revendications

1. Procédé de déshydroaromatisation non oxydative d'un courant de réactifs E contenant des composés aliphatiques en C₁-C₄, comprenant les étapes suivantes :
I. la mise en réaction du courant de réactifs E en conditions non oxydatives en présence d'un catalyseur dans une zone de réaction 1 pour former un courant de produits P contenant des hydrocarbures aromatiques,
II. la régénération du catalyseur de l'étape I d'activité réduite par le coke déposé avec un courant gazeux H contenant de l'hydrogène dans une zone de réaction 2, au moins une partie du coke déposé étant transformé en méthane et un courant gazeux M contenant du méthane se formant,
**caractérisé en ce qu'**au moins une partie du méthane formé lors de la régénération dans la zone de réaction 2 est introduit dans la zone de réaction 1.

2. Procédé selon la revendication 1, **caractérisé en ce qu'**au moins une partie du courant gazeux M formé lors de la régénération est introduite dans la zone de réaction 1.

3. Procédé selon la revendication 1 ou 2, **caractérisé en ce qu'**au moins une partie de la chaleur formée lors de la régénération du catalyseur à l'étape II dans la zone de réaction 2 est introduite dans la zone de réaction 1.

4. Procédé selon l'une quelconque des revendications 1 à 3, **caractérisé en ce que** le catalyseur est utilisé mélangé avec un matériau inerte.

5. Procédé selon l'une quelconque des revendications 1 à 4, **caractérisé en ce qu'**au moins une partie de la chaleur formée lors de la régénération du catalyseur à l'étape II dans la zone de réaction 2 est introduite directement dans la zone de réaction 1 par transfert d'au moins une partie du catalyseur régénéré de la zone de réaction 2.

6. Procédé selon l'une quelconque des revendications 1 à 5, **caractérisé en ce qu'**au moins une partie de la chaleur formée lors de la régénération du catalyseur à l'étape II dans la zone de réaction 2 est introduite directement dans la zone de réaction 1 par le courant gazeux M de la zone de réaction 2.

7. Procédé selon l'une quelconque des revendications 1 à 6, **caractérisé en ce qu'**au moins une partie de la chaleur nécessaire à l'étape I est introduite par
i) déchargement d'au moins une partie du catalyseur se trouvant dans la zone de réaction 1 ou 2 de la zone de réaction 1 ou 2,
ii) chauffage du catalyseur déchargé à une température supérieure à la température dans la zone de réaction 1, et
iii) recyclage du catalyseur chauffé dans la zone de réaction 1.

8. Procédé selon l'une quelconque des revendications 1 à 7, **caractérisé en ce que** la température à l'entrée de la zone de réaction 2 est supérieure à la température à l'entrée de la zone de réaction 1.

9. Procédé selon l'une quelconque des revendications 1 à 8, **caractérisé en ce que** la zone de réaction 1 et la zone de réaction 2 se trouvent séparées dans l'espace dans un réacteur.

10. Procédé selon l'une quelconque des revendications 1 à 8, **caractérisé en ce que** la zone de réaction 1 et la zone de réaction 2 se trouvent séparées dans l'espace dans différents réacteurs.

11. Procédé selon l'une quelconque des revendications 1 à 10, **caractérisé en ce que** la zone de réaction 1 est transformée en la zone de réaction 2 à intervalles temporels par diminution du courant de réactifs E et introduction du courant gazeux H.

12. Procédé selon l'une quelconque des revendications 1 à 11, **caractérisé en ce que** la zone de réaction 2 est transformée en la zone de réaction 1 à intervalles temporels par diminution du courant gazeux H et introduction du courant de réactifs E.

13. Procédé selon l'une quelconque des revendications 1 à 12, **caractérisé en ce que** le catalyseur dans la zone de réaction 1, dans la zone de réaction 2 ou dans les deux zones de réaction se présente sous la forme d'un lit fluidisé.

14. Procédé selon l'une quelconque des revendications 1 à 13, **caractérisé en ce que** le courant de réactifs E contient au moins 50 % en moles de composés aliphatiques en C₁-C₄.

15. Procédé selon l'une quelconque des revendications 1 à 14, **caractérisé en ce que** le courant de réactifs E contient 0,1 à 10 % en volume d'hydrogène.

16. Procédé selon l'une quelconque des revendications 1 à 15, **caractérisé en ce que** le courant gazeux H contient au moins 50 % en volume d'hydrogène.

17. Procédé selon l'une quelconque des revendications 1 à 16, **caractérisé en ce que** le catalyseur contient au moins un silicate d'aluminium et au moins un métal choisi dans le groupe constitué par Mo, W, Mn et Re.

18. Procédé selon la revendication 17, **caractérisé en ce que** le catalyseur contient au moins un métal supplémentaire choisi dans le groupe constitué par Cr, Mn, V, Zn, Zr, Ga, Cu, Ni, Co et Fe.
